# EUROPEAN PATENT APPLICATION

(11) **EP 2 324 877 A1**
(43) Date of publication of application: **25.05.2011**
(21) Application number: 10191100.6
(22) Date of filing: 12.11.2010
(51) Int. Cl.: A61M 37/00, F15B 11/00

(54) **DRIVING DEVICE, PARTICULARLY FOT TATOO, DEMOGRAPHIA AND THE LIKE MACHINES**

(30) Priority: 23.11.2009 IT MI20092058
(71) Applicant: Dell'Aquila, Luca, 26855 Lodi Vecchio (LODI) (IT); Galvan, Mauro, 20070 Cerro al Lambro (Milano) (IT)
(72) Inventor: Dell'Aquila, Luca, 26855 Lodi Vecchio (LODI) (IT); Galvan, Mauro, 20070 Cerro al Lambro (Milano) (IT)
(74) Representative: Cicogna, Franco

(57) **Abstract**

A driving device for tattoo, dermographia and the like machines comprises a hollow body encompassing a contoured piston including a piston stem and associated with a resilient element, the contoured piston including a side cut-out which, during a reciprocating movement of the piston, is alternately communicated with a pressurized air inlet hole and a pressurized air outlet hole formed on a side of the hollow body, the inlet hole communicating with a pressurized air source, thereby as pressurized air is fed through the inlet hole the piston is lowered and the resilient element is tensioned whereas, as the piston is arranged at a lowered position thereof, pressurized air in the cut-out exists thereby releasing the piston to allow it to be recovered, by the resilient element, to a starting top position therefrom a new tattoo operating cycle may be started again.

## Description

### BACKGROUND OF THE INVENTION

The present invention relates to a driving device, particularly for tattoo, dermographia and the like machines.

As is known, tattoo and dermographia machines or apparatus usually comprise electric coils operating as a driving motor assembly.

The above electric motor machines or apparatus solve some important problems related to the system allowing an operator to manually introduce a needle or several needles into a person skin.

In the above prior manual machines, the needle introduction depth depends on the operator skillness, and this manual operation, in addition to being a comparatively fatiguing one, does not provide a proper insertion of the needles into the skin.

Thus, prior electrically operated machines partially solve some of the above mentioned problems but are affected by serious drawbacks.

In particular the tattoo machine frame must be suitable to be easily disinfected and sterilized in autoclaves, as required by European and National rules related to the patient safety.

In this connection, it should be pointed out that disposable or single-use sterile needles, blister packaged dyes and other single-use and sterilizable fittings are already commercially available.

However, conventional electric coil motors cannot be sterilized in an autoclave, thereby preventing the overall tattoo apparatus from being properly sterilized and packaged, and, most importantly, preventing infective and transmissible diseases, such as AIDS, hepatitis, allergies and so on from being safely controlled.

Moreover, the provision of an electric motor assembly causes the tattoo machine weight to greatly increase, thereby the overall machine cannot be easily handled.

This latter drawback, moreover, is aggravated by the use of power supply electric conductors, which must be accurately insulated and handled.

### SUMMARY OF THE INVENTION

Accordingly, the aim of the present invention is to provide a driving or motor device, particularly for tattoo, dermographia and the like machines, which may be fully, easily and quickly disinfected and sterilized.

Within the scope of the above mentioned aim, a main object of the invention is to provide such a driving or motor device which may be easily fitted to all the frameworks, fittings, springs, needles and spare parts of conventional tattoo machines.

Another object of the present invention is to provide such a driving device which is advantageous both for the tattoo machine frame makers, the tattoo machine dealers, and, most importantly, for the tattoo operators, which will have available a sterilizable driving device, while holding their tattoo methods unaltered, with a further advantages of using customized tattoo machine frames or frameworks and all existing conventional spare parts therefor.

Another object of the present invention is to provide such a driving device which is very simple construction wise and may be made as a dermographia pen assembly to be easily used in aesthetic skin processing centers.

Yet another object of the present invention is to provide such a driving device which may be easily fitted to a reciprocating movement instrument, including one or more driving axes, such as a pressing and compacting fitting, for use in a lot of useful applications.

Yet another object of the present invention is to provide such a driving device which comprises frequency and skin depth needle penetration control simple means.

Yet another object of the present invention is to provide such a driving device which operates in a noiseless manner.

Yet another object of the present invention is to provide such a driving device which, owing to its specifically designed constructional features, is very reliable and safe in operation.

According to one aspect of the present invention, the above mentioned aim and objects, as well as yet other objects, which will become more apparent hereinafter, are achieved by a driving device, particularly for tattoo, dermographia and the like machines, characterized in that said driving device comprises a hollow body holding a contoured piston including a piston stem and associated with a resilient element, said contoured piston including a side cutout which, during a reciprocating movement of said piston, is alternately communicated with a pressurized air inlet hole and a pressurized air outlet hole formed on a side of said hollow body, said inlet hole communicating with a pressurized air source, pressurized air fed through said inlet hole causing said piston to be lowered and said resilient element to be tensioned, thereby, as the piston is at a lowered position thereof, pressurized air in said cutout exits said outlet hole thereby freeing said piston which is recovered, by said resilient element, to a starting top position therefrom a new operating cycle is started again.

### BRIEF DESCRIPTION OF THE DRAWINGS

Further characteristics and advantages of the present invention will become more apparent hereinafter from the following detailed disclosure of a preferred, though not exclusive, embodiment of the invention, which is illustrated, by way of an indicative, but not limitative, example in the accompanying drawings, where:

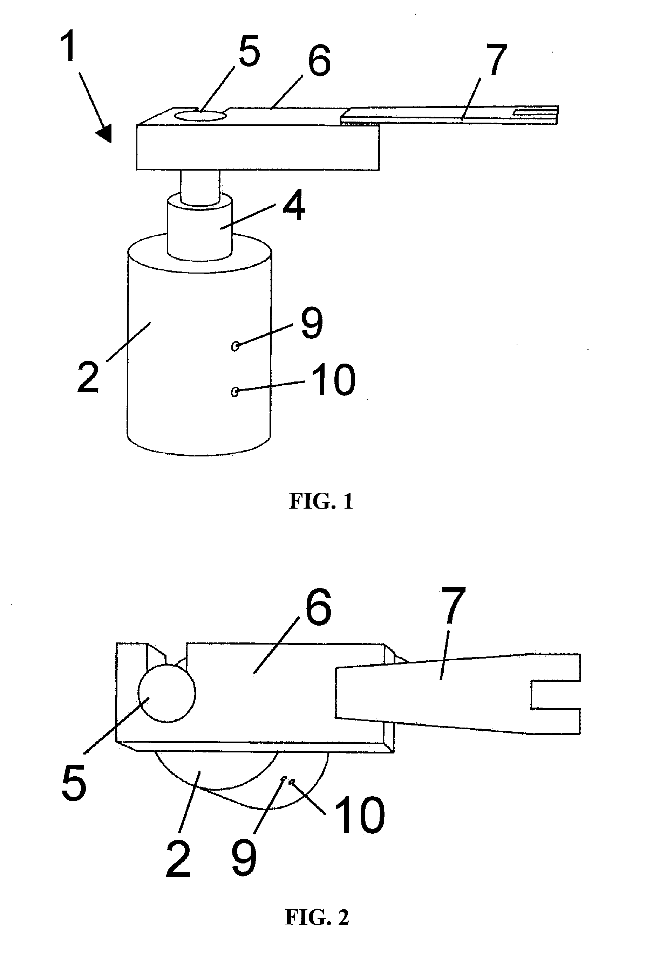

Figure 1 is a schematic perspective view of the driving or motor device according to the present invention;
Figure 2 is a top plan view of the device shown in figure 1;

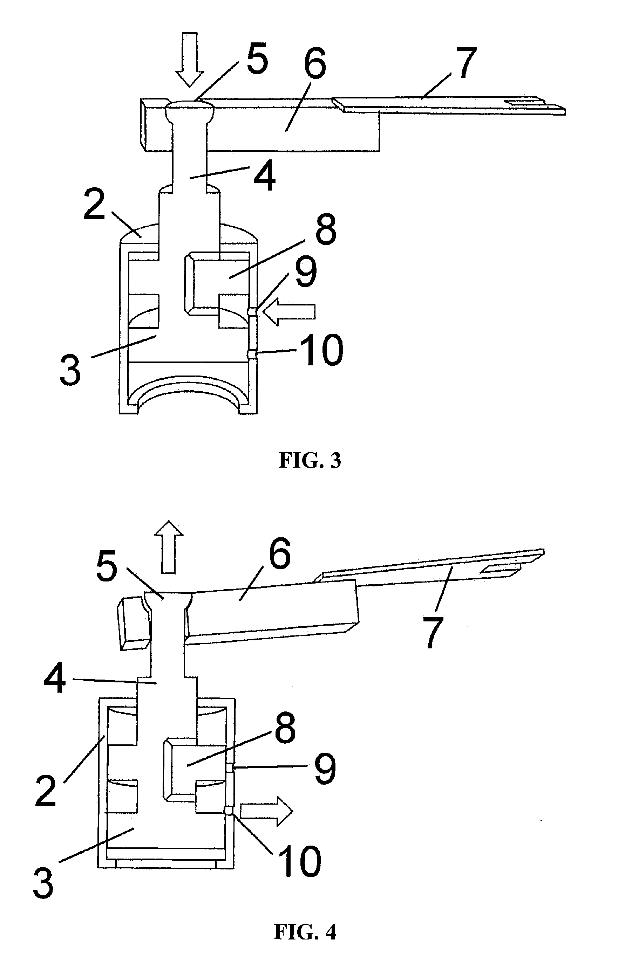

Figure 3 is a cross-sectional side elevation view showing the device of figure 2, at a pressurized air inlet condition thereof;
Figure 4 is a view similar to figure 3, but showing the inventive device in an air outlet condition thereof;
Figure 5 is a further schematic perspective view of the driving device, including a silencer apparatus, according to a further aspect of the present invention;
Figure 6 is a top plan view of the device shown in figure 5;
Figure 7 is a further cross-sectional side elevation view showing the device of figure 6 at a pressurized air inlet condition thereof ;
Figure 8 is a further cross-sectional side elevation view similar to figure 7, but showing the inventive device at an air outlet or discharging position or condition thereof;
Figure 9 is a further schematic perspective view of the inventive driving device, provided for application to a dermographia pen assembly, according to a further aspect of the present invention;
Figure 10 is yet another cross-sectional side elevation view showing the device of figure 9 at a pressurized air inlet position or condition thereof;
Figure 11 is yet another cross-sectional side elevation view similar to figure 10, but showing the inventive device at an air outlet position or condition thereof;
Figure 12 is yet another schematic perspective view of the subject driving device, specifically designed to be used for making a reciprocating movement instrument, including one or more driving axes, according to yet another aspect of the present invention;
Figure 13 is yet another cross-sectional side elevation view showing the device of figure 12 at a pressurized air inlet position or condition thereof;
Figure 14 is a view similar to figure 13, but showing the inventive device at an air outlet or discharging position or condition thereof; and
Figure 15 shows a tattoo machine or apparatus including a silenced driving or motor device according to the present invention.

### DESCRIPTION OF THE PREFERRED EMBODIMENTS

With reference to the number references of the above mentioned figures, the driving or motor device, particularly for tattoo, dermographia and the like machines, according to the present invention, which has been generally indicated by the reference number 1, comprises an hollow body 2, therein a contoured or shaped piston 3, having a piston stem 4 is arranged.

Said piston stem 4 comprises a piston stem foot or end portion 5, associated with a movable body 6 which is integral or made rigid with a reed or blade spring 7.

The piston 3 further comprises a side cut-out 8 which, during the reciprocating movement of said piston, will be alternately communicated with an inlet hole 9 and an outlet hole 10 formed through a side of said hollow body 2.

Said inlet hole 9, in particular, is communicated with a pressurized air source, not shown in the figures.

Thus, as pressurized air is fed through the inlet hole 9, it will cause the piston 3 to be lowered, while tensioning the blade or reed spring 7.

With the piston 3 at the lowermost position thereof, the pressurized air held in the cut-out portion 8 will exit the outlet hole 10, thereby freeing the piston 3 which will be cause to return by the reed or blade spring 7, to the top position thereof, thereby recovering the operating cycle again.

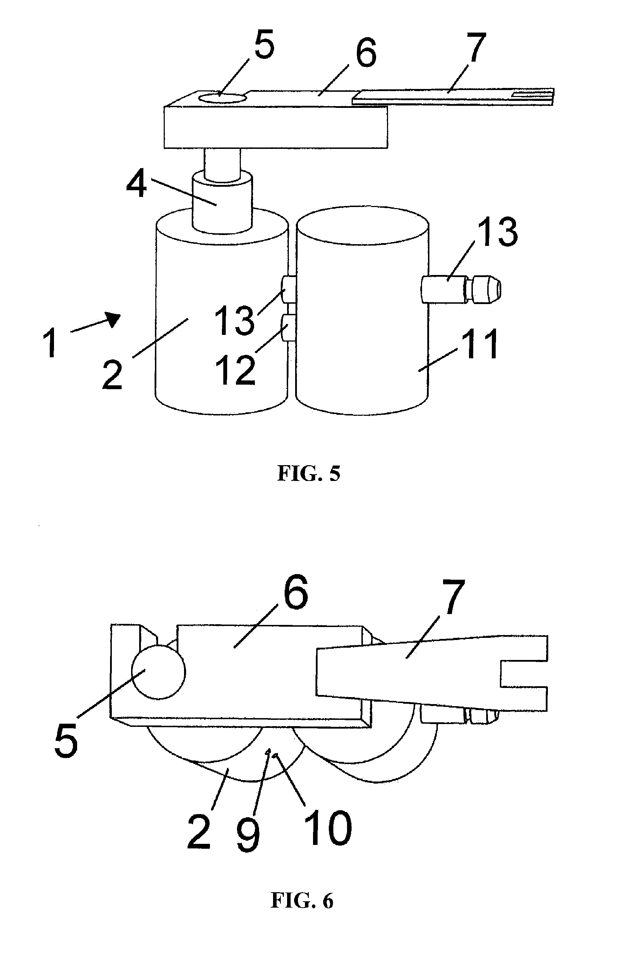

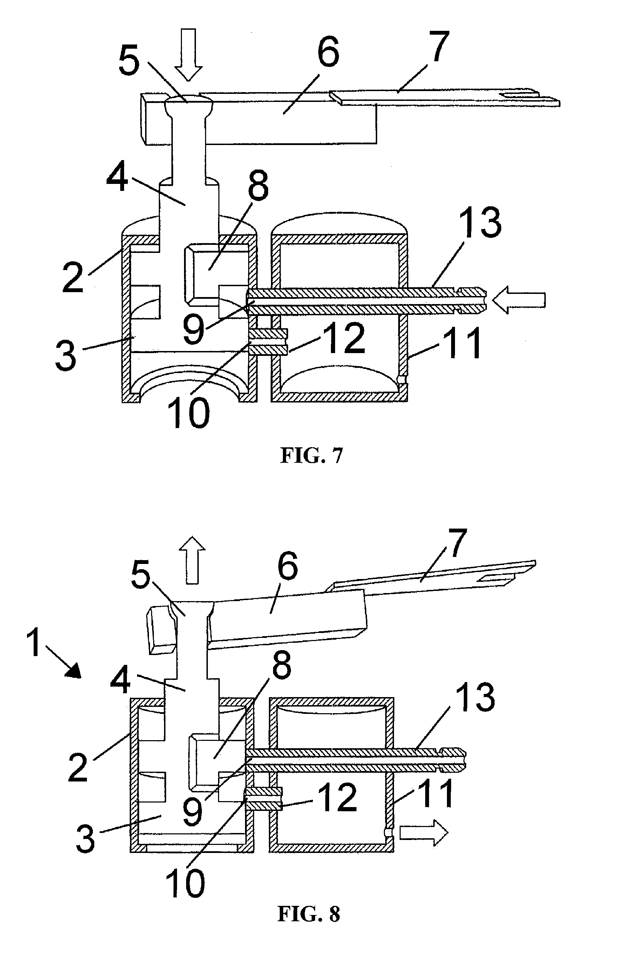

Figures 5-8 show a driving or motor device including a silencer apparatus comprising a second hollow body 11 which is connected to the outlet or discharging hole 18 through an outlet or discharging duct 12.

An inlet duct 13, passing through the second hollow body 11, without communicating therewith, in order to properly feed pressurized air generated by a pressurized air source, not shown in the figures, to the inlet hole 9 of the hollow body 2 is herein provided.

The operation of the silenced driving or motor device is fully analogous to that which has been thereinabove disclosed, with the single difference that pressurized air exiting the outlet hole 10 will enter the second hollow body 11 thereby damping sound waves generated by an intermittent pressurized air discharging.

In this connection it should be pointed out that the pressurized air driving or motor device 1 according to the present invention may be fully and easily disinfected and sterilized, and may replace, for all the intended applications or operations thereof, a prior electric coil motor assembly.

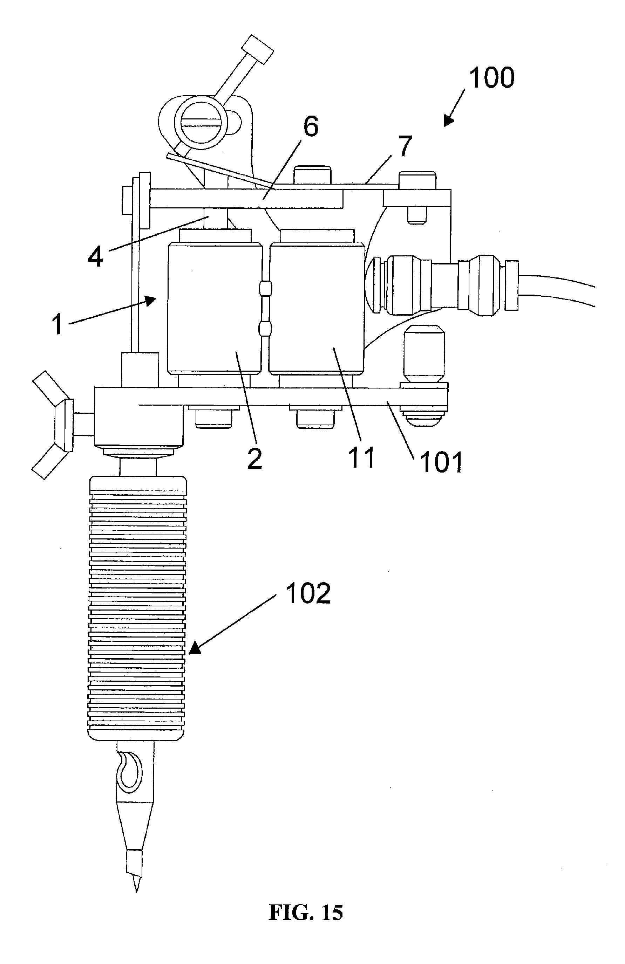

Figure 15 shows an exemplary embodiment for applying the pressurized air driving or motor device 1, according to the present invention, to a tattoo machine or apparatus 100 comprising a machine supporting framework 101, which is removably associated with a needle assembly 102.

The needle assembly 102, in turn, is operatively associated with the motor or driving device movable body 6, thereby actuating with a reciprocating movement said needle assembly.

The driving or motor device according to the present invention may be easily fitted to all electric coil operated machine tattoo frameworks, as well as to all fittings, springs, needles and spare parts therefor.

Moreover, the driving device according to the present invention provides great advantages both for prior tattoo machine framework makers, which will be able of preserving their preferred configuration, and for the tattoo machine dealers, which may be able of using those same prior fittings and, at most importantly, for the tattoo operators which will have available an easily and quickly sterilizable driving or motor device, while holding their tattoo method unaltered, since they will use their existing customized tattoo machine frameworks and existing prior spare parts therefor.

In this connection it should be moreover pointed out that the driving device according to the present invention also comprises operating frequency control systems, based on the air pressure, as well as an adjusting system for properly adjusting the skin penetration needle depth, depending on the reed or blade spring features or spring rate.

Thus, the driving motor according to the present invention may be easily and quickly fitted, owing to its very simple construction, for making a dermographia pen assembly, of a type already used in aesthetic centers.

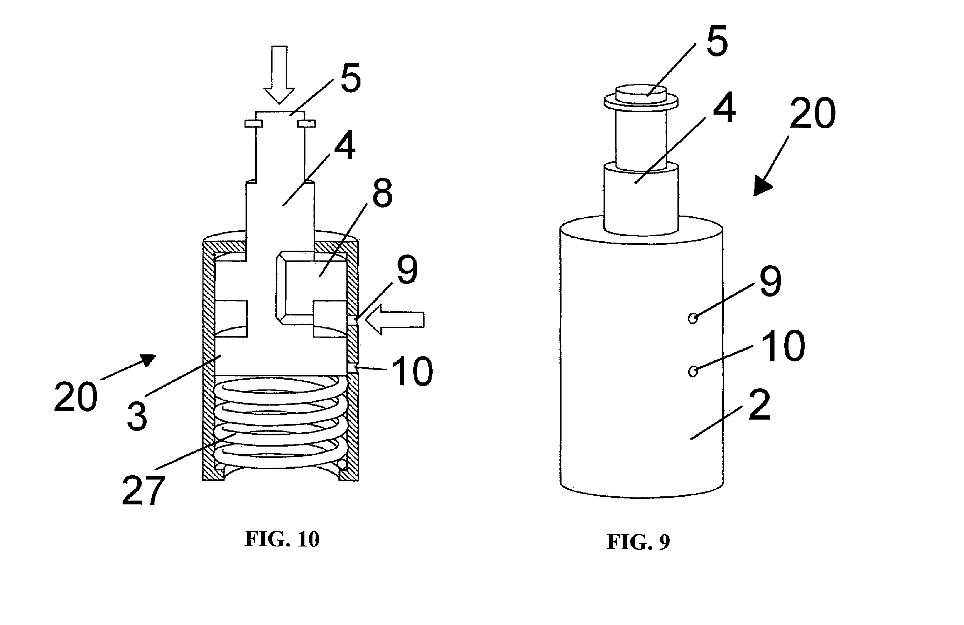

Figures 9-11 show a further embodiment of a motor or driving device, generally indicated by the reference number 20, specifically designed for dermographia pen assemblies, in which, as a replacement of the flexure or bending spring provided in prior tattoo machines, comprises moreover a coil spring 27, or other resilient or elastic memory means, applied to a bottom portion of the hollow body 2.

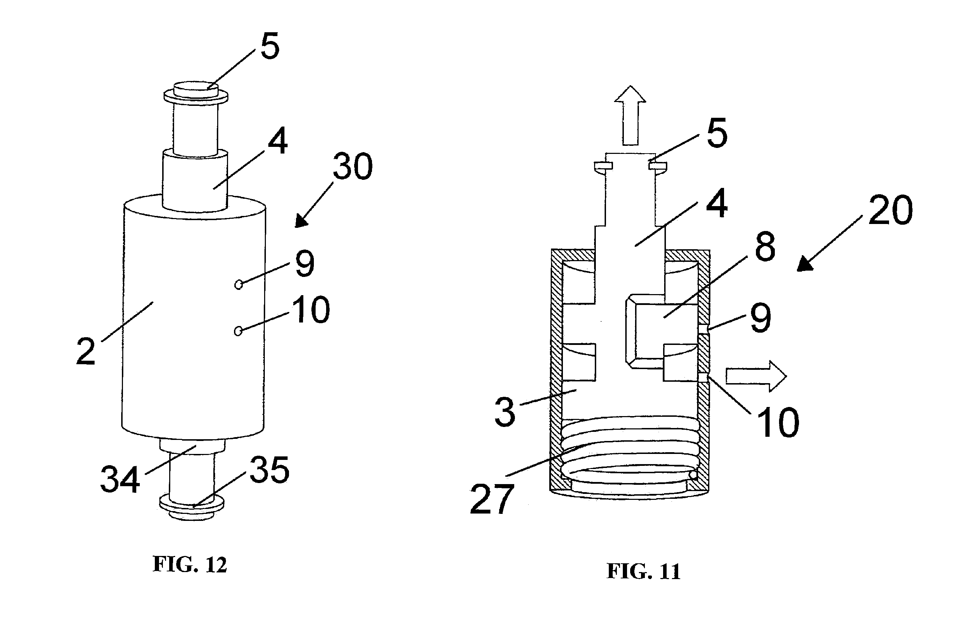

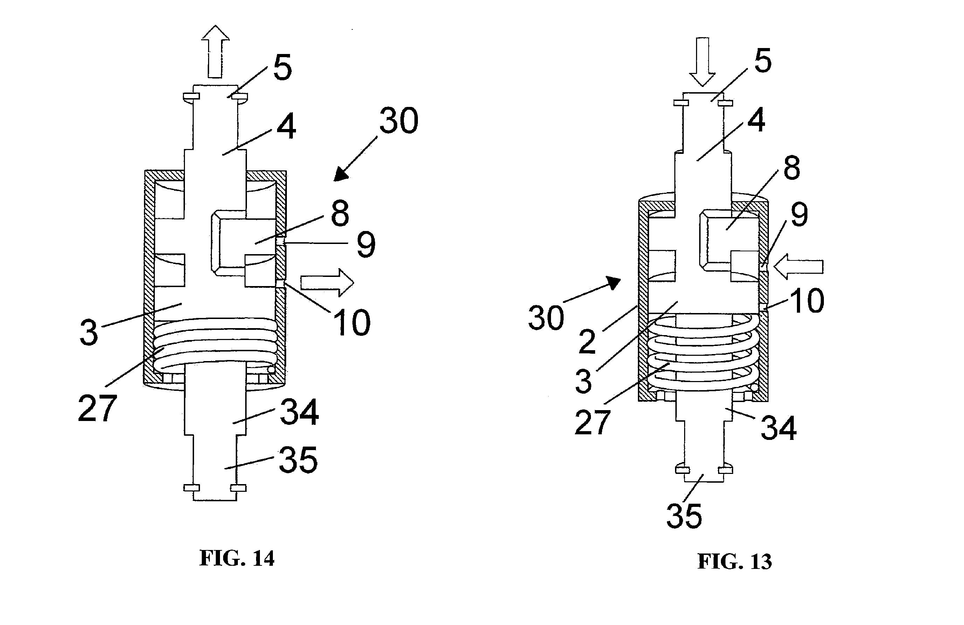

Figures 12-14 shows a further embodiment of the inventive driving device, generally indicated by the reference number 30, which may be used for making a reciprocating movement instrument, including either one or more instrument axes, for example a pressing and compacting device, to be used in a lot of different operative applications.

The driving device 30 comprises, in this case, in addition to the stem element 4 including the stem element foot portion 5, a second operating stem 34, including a related operating foot portion 35 arranged coaxially with respect to the coil spring 27.

It has been found that the invention fully achieves the intended aim and objects.

In fact, the invention has provided a pressurized air driving or motor device, which allow prior tattoo machines to be easily and quickly sterilized, while efficiently replacing the conventional prior electric coil motor assembly.

In practicing the invention, the used materials, as well as the contingent size and shapes, can be any, depending on requirements.

## Claims

1. A driving device, particularly for tattoo, dermographia and the like machines, **characterized in that** said driving device comprises a hollow body holding a contoured piston including a piston stem and associated with a resilient element, said contoured piston including a side cutout which, during a reciprocating movement of said piston, is alternately communicated with a pressurized air inlet hole and a pressurized air outlet hole formed on a side of said hollow body, said inlet hole communicating with a pressurized air source, pressurized air fed through said inlet hole causing said piston to be lowered and said resilient element to be tensioned, thereby, as the piston is at a lowered position thereof, pressurized air in said cutout exits said outlet hole thereby freeing said piston which is recovered, by said resilient element, to a starting top position therefrom a new operating cycle is started again.

2. A driving device, according to claim 1, **characterized in that** said stem comprises a stem foot associated with a movable body which is rigid with a blade spring.

3. A driving device, according to claim 1, **characterized in that** said device comprises a silencer apparatus.

4. A driving device, according to claim 3, **characterized in that** said silencer apparatus is constituted by a second hollow body.

5. A driving device, according to claim 4, **characterized in that** said second hollow body of said silencer apparatus is coupled to said outlet hole through an outlet duct.

6. A driving device, according to claim 5, **characterized in that** said silencer apparatus second hollow body is coupled to said outlet hole through an outlet duct, an inlet duct passing through said second hollow body without communicating with said hollow body supplying pressurized air from a pressurized air source to said inlet hole of said second hollow body.

7. A driving device, according to claim 1, **characterized in that** said driving device is applied to a tattooing machine comprising a supporting framework to which a needle assembly is removably coupled, said needle assembly being operatively associated with the movable body of said driving device, for reciprocately driving said needle assembly.

8. A driving device, according to claim 1, **characterized in that** said device further comprises a pressurized air operating beating frequency control system and an adjusting system for adjusting a penetrating force of said needle into a user's skin, said penetrating force varying depending on a stiffness of said resilient element.

9. A driving device, according to claim 1, **characterized in that** said device comprises a resilient coil spring or other suitable resilient memory means applied to a bottom of said hollow body, said driving device and said coil spring providing an assembly adapted to be used in a dermographic pen.

10. A driving device, according to claim 1, **characterized in that** said driving device comprises a second stem, including a second stem foot, and being arranged coaxially with said coil spring, said driving device and second stem assembly being adapted to be used for making a reciprocating tool having one or more reciprocating axes, such as a striker and a compacting assembly.
